Europäisches Patentamt

European Patent Office

Office européen des brevets

(11) Publication number: **0 179 416**
A2

# EUROPEAN PATENT APPLICATION

(21) Application number: 85113261.3

(22) Date of filing: 18.10.85

(51) Int. Cl.⁴: **A 61 K 7/48**

(30) Priority: 22.10.84 US 663276

(43) Date of publication of application:
30.04.86 Bulletin 86/18

(84) Designated Contracting States:
AT CH DE GB LI

(71) Applicant: PLOUGH, INC.
3030 Jackson Avenue
Memphis Tennessee 38151(US)

(72) Inventor: Suss, Harold
7586 Blackberry Farm Road
Germantown Tennessee 38138(US)

(72) Inventor: Ner, Virginia Feliciano
5140 Steuben Drive
Memphis Tennessee 38134(US)

(74) Representative: von Kreisler, Alek, Dipl.-Chem. et al,
Deichmannhaus am Hauptbahnhof
D-5000 Köln 1(DE)

(54) Long wear cosmetics.

(57) A composition adaptable for use as a long wear cosmetic or cosmetic base containing a pentaerythritol tetra ($C_{20}$-$C_{24}$) aliphatic hydrocarbon carboxylate, particularly pentaerythritol tetrabehenate is disclosed. Various uses of the compositions of the invention, e.g., as long wear eye cream, long wear eye stick, a long wear foundation, a long wear lipstick or lip cream, or as a long wear eyeliner or mascara are also disclosed.

EP 0 179 416 A2

## LONG WEAR COSMETICS

This invention is a composition adaptable for use as a long wear cosmetic or cosmetic base. The long wear compositions of this invention are useful as foundations, eye creams, eyeliners, eyesticks, mascaras, lipsticks and lip creams.

As used throughout the present specification and claims the term "conventional" as an adjective defining a cosmetic, cosmetic base, or other composition is intended to mean a composition that does not contain pentaerythritol tetra ($C_{20}$-$C_{21}$) aliphatic hydrocarbon carboxylate.

The compositions of this invention contain from about 2 to about 25 weight percent of a pentaerythritol tetra ($C_{20}$-$C_{24}$) aliphatic hydrocarbon carboxylate in conventional cosmetic or cosmetic base compositions. This invention is based on the finding that the pentaerythritol tetra ($C_{20}$-$C_{24}$) aliphatic hydrocarbon carboxylate provides surprising and highly desirable long-wear properties to make-up compositions.

The compositions of this invention may be formulated as a long wear eye cream, eyeliner, eyestick, foundation, lipstick, lip cream or mascara. The present invention also provides a method for extending the effective wear time of a cosmetic which comprises admixing pentaerythritol tetra ($C_{20}$-$C_{24}$) aliphatic

hydrocarbon carboxylate with a conventional cosmetic so that the resulting composition contains from about 2 to 25 weight percent pentaerythritol tetra $(C_{20}-C_{24})$ aliphatic hydrocarbon carboxylate.

We have discovered that incorporation of a sufficient amount of a pentaerythritol tetra $(C_{20}-C_{24})$ aliphatic hydrocarbon carboxylate into a conventional cosmetic base or cosmetic provides long wear lip and eye wear products having advantageous properties compared to commercial products, e.g., lip creams such as "Lip Prime" (Nat Robbins) or "LIP-FIX" cream (Elizabeth Arden) or eye creams such as "EYE-FIX" primer (Elizabeth Arden). The compositions of the present invention adapted for use as a lip cosmetic base and applied to the lips were found effective in controlling, for longer wear times, bleeding and feathering of lipstick applied thereon without interfering with lipstick luster. The compositions of the present invention adapted for use as an eye cosmetic base and applied to the eyelids were found to extend the wear time of eye shadow wear. The eye cream compositions of the present invention are deposited on the eyelids as a tight, even moisturizing film, thereby allowing makeup to be applied thereon smoother and to stay on longer while effectively controlling creasing, color fading and change on the eyelid for longer time periods than conventional commercial compositions.

The cosmetic and cosmetic base compositions of the present invention contain from about 2 to about 25 weight percent, preferably about 5 to 20 weight percent of a pentaerythritol tetra $(C_{20}-C_{24})$ aliphatic hydrocarbon carboxylates. Typical suitable pentaerythritol tetra $(C_{20}-C_{24})$ aliphatic hydrocarbon carboxylates include straight and branched chain $(C_{20}-C_{24})$ alkanoates derived from aliphatic monocarboxylic acids (fatty acids) such as arachidic $(C_{20})$,

0179416

heneicosanoic ($C_{21}$), behenic (or docosanoic) ($C_{22}$), tricosanoic ($C_{23}$) and lignoceric (or tetracosanoic) ($C_{24}$) as well as mono unsaturated alkenoates such as $\underline{cis}$- and $\underline{trans}$-$\Delta^{13}$-docosenoic ($C_{23}$) as well as isoerucic (a mixture of $\Delta^{14}$-docosenoic, ceteloic ($\Delta^{11}$-docosenic) and nervonic ($\Delta^{15}$-tetracosenoic). Surprisingly, the tetra ester of pentaerythritol and $C_{18}$ fatty acids (stearic and oleic acids) do not provide the superior advantages of those included within the scope of the compositions of the present invention. The preferred pentaerythritol tetra($C_{20}$-$C_{24}$) aliphatic hydrocarbon carboxylate is pentaerythritol tetrabehenate.

The cosmetic compositions of the present invention may be in liquid, solid, or semi-solid form and can be either aqueous or anhydrous. The aqueous compositions adaptable for use as long wear cosmetics and cosmetic bases in the form of an eye cream, a foundation, a mascara or a lip cream, are generally composed of, in addition to the effective amount of a pentaerythritol tetra($C_{20}$-$C_{24}$) aliphatic hydrocarbon carboxylate, an oil-in-water emulsion as found in conventional cosmetic bases. The oil part contains as a major ingredient one or more vegetable, animal and mineral oils, fats, silicones, and waxes, polymers, higher carboxylic acid esters and, as a minor component, color pigments, pearlizing agents, mica, perfume, humectants, sunscreens, surfactants, preservatives, suspending agents, bodying agents and/or antioxidants.

The water used in the aqueous compositions of the present invention is preferably microbially-free, deionized water.

Typical suitable waxes include ozokerite, lanolin alcohol, paraffin wax, bayberry wax, trihydroxystearin, lanolin wax, beeswax, Candellila wax, microcrystalline wax, Carnauba wax, cetyl alcohol, stearyl alcohol, spermaceti, cocoa butter, fatty acids of lanolin, mono-, di- and triglycerides which are solid at 25°C, fatty esters which are solid at 25°C, silicone waxes such as methyloctadecane-oxypolysiloxane and poly (dimethylsiloxy) stearoxysiloxane, stearyl monoethanolamide, rosin and its derivatives such as the abietates of glycol and glycerol, hydrogenated oils solid at 25°C, and sucroglycerides.

Typical suitable oils include paraffin oil, lanolin, purcellin oil, perhydrosqualene, hydrogenated lanolin, hydroxylated lanolin, acetylated lanolin, petrolatum, castor oil, polybutene, odorless mineral spirits, sweet almond oil, avocado oil, calophyllum oil, ricin oil, horse oil, hog oil, olive oil, castor oil, mineral oil having a boiling point between 310° and 410°C, silicone oil such as dimethylpolysiloxane and cyclomethicone, linoleic alcohol, linolenic alcohol, oleyl alcohol, the oil of cereal germs such as the oil of wheat germ, isopropyl lanolate, isopropyl palmitate, isopropyl myristate, butyl myristate, cetyl myristate, myristyl myristate, myristyl lactate, hexadecyl stearate, butyl stearate, decyl oleate, acetyl glycerides, the octanoates and benzoates of $(C_{12}-C_{15})$ alcohols, the octanoates and decanoates of alcohols and polyalcohols such as those of glycol and glycerol, ricinoleates of alcohols and of poly alcohols such as those of cetyl alcohol or isostearyl alcohol, isocetyl lanolate, isopropyl adipate, hexyl laurate and octyl dodecanoate. It is preferred to employ a mixture of these oils for their different functions: for example oleyl alcohol is a penetrant and color vehicle; castor oil is a color dispersing agent; mineral oil and cyclomethicone are emollients and moisturizers.

Typical suitable sunscreens include titanium dioxide, and lower alkyl para-methoxycinnamates, and para-aminobenzoates.

Typical suitable antioxidants include propyl, octyl and dodecyl esters of gallic acid, butylated hydroxy anisole, butylated hydroxy toluene and nordihydroguaiaretic acid.

Typical suitable pearlizing agents include bismuth oxychloride, titaniumdioxide-coated mica and crystals of guanine. Generally, the pearlizing agents are dispersed in an oily vehicle.

Typical suitable preservatives include the lower alkyl esters of para-hydroxybenzoates especially the methyl, ethyl, propyl, butyl, and isobutyl esters and mixtures thereof, imidazolidinyl urea, or diazolidinyl urea.

Typical suitable humectants include butylene glycol, propylene glycol, reticulin (the protein) and vitamin E.

Typical suitable surfactants include nonionic, anionic and cationic surfactants such as sorbitan stearate, Oleth-3 phosphate and Quaternium-18, respectively.

Polymers may be used to achieve one or more desirable effects. They may, for example, serve as suspending aids, emulsion stabilizers, emulsification aids, binders, thickeners or film formers, or may provide water resistance, water proofing or gloss. Typical suitable polymers include methylcellulose, hydroxyethylcellulose; vinyl polymers and copolymers, e.g., polyvinyl pyrrolidone (PVP) homopolymers and PVP copolymers such as PVP/eicosene copolymer, PVP/hexadecene copolymer; polyvinylacetate (PVA) homopolymers and PVA copolymers; ethylene oxide homopolymers/copolymers and derivatives; and acrylic polymers, e.g., acrylic/acrylate copolymers.

Typical suitable bodying agents include Carbomers especially Carbomer 934P, which are polymers of acrylic acid crosslinked with a polyfunctional agent and available from B.F. Goodrich Chemical Co under the Carbopol tradename; methylcellulose, hydroxyethylcellulose and polyacrylates.

Agents suitable for suspending pigments include magnesium aluminum silicate, polybutene, and Benton Gels, the tradename of N.L. Chemicals, Div. of NL Industries, for a series of modified hectorites (one of montmorillonite minerals that are the principal constituents of bentonite clay).

The compositions of the present invention used in the form of lipstick and eye wear stick may be formulated without water and stiffened to the desired consistency with waxes which also raise the melting point and improve the physical stability.

The compositions of the present invention adaptable for use as cosmetics also include one or more color pigments or coloring materials, e.g., organic dyes and inorganic pigments which are usually dispersed in an oily vehicle.

Typical suitable dyes employed in the cosmetic compositions of the present invention are the U.S. Government certified colors, both Drug and Cosmetic grade and Food, Drug and Cosmetic grade, e.g., D&C reds, oranges, yellows and blues. The pigments employed are generally inorganic pigments such as iron oxides, titanium dioxides, ultramarines, iron sulfides, or other conventional pigments approved for cosmetic use. The dyes and pigments are preferably employed in an amount ranging from about 1% to about 10% by weight of the formulation with about 2% to about 4% being more preferred.

When the cosmetics and cosmetic bases of the present invention are anhydrous, a volatile component is generally used instead of water. Typically suitable volatile components include odorless petroleum distillates, e.g., Shell Sol 71 available from Shell Chemical Co., odorless oil of turpentine, white spirits, volatile silicone oils, e.g., cyclomethicone (cyclic dimethyl polysiloxane), deca- or octa-methylcyclopentasiloxane, ethanol and/or isopropyl alcohol (which also function as a viscosity control agent) and the like.

A typical eyeliner or mascara in the anhydrous form may contain, in addition to the effective amount of a pentaerythritol tetra($C_{20}$-$C_{24}$) aliphatic hydrocarbon carboxylate, one or more volatile components, e.g. isopropyl alcohol, one of more inorganic polymers for thickness, e.g., montmorillonite clay, one or more waxes as viscosity aids, as well as antioxidants, preservatives and processing aids, e.g., dimethicone which is a mixture of fully methylated siloxane polymers end-blocked with trimethylsiloxy units.

The following examples illustrate the invention. Definitions of the ingredients used may be found in the CTFA Cosmetic Ingredients Dictionary, published by The Cosmetic, Toiletry, and Fragrance Association, Inc., 1110 Vermont Avenue, NW, Washington, D.C. 20005, U.S.A. If any ingredients used in these examples are not available, substitutions may be made, since it is believed that the incorporation of pentaerythritol tetra ($C_{20}$-$C_{24}$) aliphatic hydrocarbon carboxylate will improve the wear time when formulated into any conventional cosmetic composition.

-8-

EXAMPLE I

LONG WEAR EYE CREAM

| Ingredients | Parts by Wgt. | |
|---|---|---|
| Deionized water | 71.55 | A |
| Carbomer 934P[1] | 0.20 | |
| Propylene Glycol | 4.00 | B |
| Methyl p-Hydroxybenzoate | 0.20 | |
| Cetyl Alcohol | 1.50 | C |
| Sorbitan Stearate | 1.50 | |
| Pentaerythritol tetrabehenate | 6.00 | |
| Oleth-3 Phosphate[2] | 0.50 | |
| Polawax[3] | 5.00 | |
| n-Propyl p-Hydroxybenzoate | 0.10 | |
| Cyclomethicone[4] | 4.00 | D |
| Triethanolamine | 0.55 | E |
| Deionized Water | 1.00 | |
| Diazolidinyl Urea | 0.25 | F |
| Trisodium Ethylene Diamine Tetraacetic Acid | 0.10 | |
| Water | 2.00 | |
| Saccharide Isomerate | 0.50 | G |
| Reticulin (the protein) | 1.00 | |
| Vitamin E | 0.05 | |

[1] A polymer of acrylic acid crosslinked with a polyfunctional agent and available under tradename Carbopol 934P from B.F. Goodrich Chem. Co., Cleveland, Ohio, U.S.A.

[2] A complex mixture of esters of phosphoric acid and the propylene glycol ether of oleyl alcohol available under the tradename Crodafos N.3 Acid from Croda, Inc., N.Y., N.Y., U.S.A.

[3] Reaction product of higher fatty alcohols and ethylene oxide available from Croda, Inc., N.Y., N.Y., U.S.A.

[4] A cyclic dimethyl polysiloxane compound having the

formula $\boxed{-[Si(CH_3)_2-O]_n}$ wherein n

averages from 3 and 6.

Prepare by adding to a homogeneous mixture A successively the ingredients in B, C, D, E, F and G using mixing and heat where appropriate until a homogenous dispersion of all ingredients is achieved.

## EXAMPLE II
### LONG WEAR EYE CREAM

| Ingredients | Parts by Wgt. | |
|---|---|---|
| Deionized water | 68.29 | A |
| Talc | 3.00 | |
| Triethanolamine | 0.35 | |
| Glycerine | 2.00 | |
| Trisodium Ethylenediamine Tetraacetic Acid | 0.010 | |
| Cetyl Alcohol | 1.00 | B |
| Sorbitan Stearate | 1.50 | |
| Pentaerythritol Tetrabehenate | 6.00 | |
| Oleth-3 Phosphate | 0.50 | |
| n-Propyl p-Hydroxybenzoate | 0.10 | |
| Polawax | 5.00 | |
| Octyl Palmitate | 1.00 | |
| Cyclomethicone | 4.00 | C |
| Carbomer 934P | 0.16 | D |

| Ingredients | Parts by Wgt. | |
|---|---|---|
| Propylene Glycol | 3.00 | E |
| Methyl p-Hydroxybenzoate | 0.20 | |
| Diazolidinyl Urea | 0.25 | F |
| Deionized Water | 2.00 | |
| Saccharide Isomerate | 0.50 | G |
| Reticulin (the protein) | 1.00 | |
| Vitamin E | 0.005 | |

Prepare by adding successively the ingredients in B to G to a homogenous mixture of ingredients in A until a homogeneous dispersion is achieved.

## EXAMPLE III
### LONG WEAR EYE LIP CREAM

| Ingredients | Parts by Wgt. | |
|---|---|---|
| Deionized Water | 63.80 | A |
| Carbomer 934P | 0.20 | |
| Glycerine | 4.00 | |
| Methyl p-Hydroxybenzoate | 0.20 | |
| Butylene Glycol | 2.00 | |
| Sorbitan Stearate | 3.00 | B |
| Pentaerythritol Tetrabehenate | 8.00 | |
| Polawax | 5.00 | |
| Cetyl Alcohol | 1.00 | |
| Oleth-3 Phosphate | 1.50 | |
| Octyl Palmitate | 1.00 | |
| n-Propyl p-Hydroxybenzoate | 0.10 | |
| Octyl p-Methoxycinnamate | 2.25 | |
| Cyclomethicone | 4.00 | C |
| Triethanolamine | 0.70 | D |

| Ingredients | Parts by Wgt. | |
|---|---|---|
| | | E |
| Trisodium Ethylene Diamine Tetraacetic Acid | 0.10 | |
| Imidazolidinyl Urea | 0.40 | |
| Deionized Water | 2.00 | |
| Saccharide Isomerate | 0.50 | F |
| Reticulin (the protein) | 1.00 | |
| Vitamin E | 0.05 | |

Prepare by mixing the water and Carbomer 934P of A until well dispersed. Heat to 80°C. Add rest of ingredients in A, and maintain A at 80°C. Heat the ingredients of oil phase (B) and (C) until homogenous. Slowly add B and C to A at 80°C. Mix for 3-5 minutes until a homogenous mixture forms. Cool to 70°C and add D. Cool to 60°C and add E. Cool to 40°C and add F. Cool to 35°C.

## EXAMPLE IV
### LONG WEAR LIPSTICK

| Ingredients | Parts by Wgt. | |
|---|---|---|
| Paraffin NF | 3.50 | A |
| Ozokerite 170 NF | 3.50 | |
| Microcrystalline wax | 2.12 | |
| Candellila wax | 3.50 | |
| Lanolin, Anhyd. USP | 5.00 | |
| Castor Oil | 14.71 | B |
| Mica | 6.00 | |
| Oleyl Alcohol | 10.00 | |
| Acetylated Lanolin | 9.62 | |
| Polybutene | 18.00 | |

| Ingredients | Parts by Wgt. | |
|---|---|---|
| Myristyl Lactate | 8.70 | |
| Trihydoxystearin | 2.60 | |
| n-Propyl p-Hydroxybenzoate | 0.10 | C |
| Butylated Hydroxyanisole | 0.10 | |
| Methyl p-Hydroxybenzoate | 0.10 | |
| Pentaerythritol Tetrabehenate | 8.00 | |
| Octyl p-Methoxycinnamate | 2.25 | |
| Drometrizole | 0.10 | |
| Mixture of $C_{10}$-$C_{30}$ alkanoate esters of Sterol | 2.00 | |

Prepare by mixing the castor oil of Part B with the mica. Pass the mixture so-formed twice through a triple roller mill. Melt all the wax ingredients (Part A) in a steam-jacketed stainless steel kettle equipped with a double motion agitator at a temperature of 85°C and mix until the waxes form a homogenous mixture. Add the mixture of castor oil and mica and the other ingredients of Parts B and C to the homogenous mixture of waxes. Heat the resulting mixture to a temperature of 78°-82°C., while continuously mixing until a uniform dispersion is achieved. The dispersion so formed is poured into molds while maintaining the temperature at 78°-82°C.

The product of Example IV may be used as a base to improve the wear time of conventional lipsticks. Of course, colorants can be added if the product is to color the lips.

-13-

EXAMPLE V

LONG WEAR EYE STICK

| Ingredients | Parts by Wgt. | |
|---|---|---|
| Pentaerythritol Tetrabehenate | 25.0 | A |
| Myristyl Myristate | 2.0 | |
| Stearyl Stearate | 15.0 | |
| Polybutene | 20.0 | |
| Microcrystalline Wax | 7.0 | |
| Myristyl Lactate | 15.6 | |
| Acetylated Lanolin | 5.0 | |
| Dipropylene Glycol | 1.0 | B |
| Methyl p-Hydroxybenzoate | 0.1 | |
| n-Propyl p-Hydroxybenzoate | 0.1 | |
| n-Butyl p-Hydroxybenzoate | 0.1 | |
| Butylated Hydroxyamisole | 0.1 | |
| Mica | 9.0 | C |

Prepare by melting all ingredients in Part A in a steam-jacketed stainless steel kettle equipped with double motion agitator at a temperature of 85°C, and mixing until they completely dissolve. Add ingredients in Part B and continue mixing until a homogenous mixture is formed. Add the mica (Part C) to the mixture of A and B and continue the mixing until a uniform dispersion is achieved. The dispersion is poured onto molds while maintaining the temperature at 80-85°C.

0179416

-14-

<u>EXAMPLE VI</u>
<u>LONG WEAR EYE STICK</u>

| Ingredients | Parts by Wgt. | |
|---|---|---|
| Pentaerythritol Tetrabehenate | 25.0 | A |
| Myristyl Myristate | 3.1 | |
| Stearyl Stearate | 15.0 | |
| Polybutene | 20.0 | |
| Microcrystalline Wax | 2.0 | |
| Myristyl Lactate | 13.0 | |
| Acetylated Lanolin | 5.0 | |
| Dipropylene Glycol | 1.0 | B |
| Methyl p-Hydroxybenzoate | 0.1 | |
| n-Propyl p-Hydroxybenzoate | 0.1 | |
| n-Butyl p-Hydroxybenzoate | 0.1 | |
| Butylated Hydroxyanisole | 0.1 | |
| Mica | 10.0 | C |
| TiO$_2$, 50 weight percent in Castor Oil[1] | 0.5 | |

Prepare by following the procedure of Example V except that the titanium dioxide in castor oil is added with the mica.

<u>EXAMPLE VII</u>
<u>ANHYDROUS LONG-WEAR EYELINER</u>
<u>AND/OR MASCARA</u>

| Ingredients | Parts by Wgt. | |
|---|---|---|
| Beeswax White USP | 7.00 | A |
| Pentaerythritol Tetrabehenate | 2.00 | |
| Butylated Hydroxyanisole | 0.10 | |
| n-Butyl p-Hydroxybenzoate | 0.30 | |
| n-Propyl p-Hydroxybenzoate | 0.13 | |
| Dimethicone[1] | 0.10 | |

-15-

| Ingredients | Parts by Wgt. | |
|---|---|---|
| Odorless Petroleum Distillate | 8.50 | B |
| Bentone Gel SS-71[2] | 54.85 | |
| Black Iron Oxides | 20.00 | C |
| Isopropyl Alcohol | 7.00 | D |

[1] A mixture of fully methylated linear siloxane polymers end-blocked with trimethylsiloxy units, i.e., $(CH_3)_3SiO[-Si(CH_3)_2-O]_n-Si(CH_3)_3$.

[2] Tradename for a mixture of petroleum distillate and Quaternium-18 Hectorite[3] and propylene carbonate available from NL Chem. Div. of NL Industries, Highstown, N.J., U.S.A.

[3] A reaction product of Quaternium-18[a] and Hectorite[b]

(a) a quanternary ammonium salt that generally conforms to the formula $[(R)_2(CH_3)_2N^+]Cl$ wherein R represents hydrogenated tallow fatty radicals.

(b) one of montmorillonite minerals that are the principal constituents of bentonite clay.

Prepare by melting all the ingredients in A in a stream-jacketed stainless steel kettle equipped with double motion agitator at a temperature of 80°C and continue mixing until they completely dissolve. Add ingredients in B until a uniform dispersion is achieved. Hold temperature of the dispersion at 80°C and add thereto the ingredient in C. Continue mixing for 30 minutes while maintaining the dispersion at 80°C. Cool the dispersion to 30°C and add the ingredient in D. Mix slowly for 5 minutes.

## EXAMPLE VIII
### OIL-IN-WATER LONG WEAR EYELINERS
### AND/OR MASCARAS

| Ingredients | Compositions in Parts by Weight | | |
|---|---|---|---|
| | i | ii | |
| Deionized Water | 35.30 | 35.95 | A |
| Acrylates Copolymer[1] | 30.00 | 30.00[2] | |
| Propylene Glycol | 6.00 | 3.00 | |
| Hydroxyethylcellulose | 0.50 | .15 | |
| Methylpara-hydroxybenzoate | 0.45 | 0.45 | |
| Ammonium Hydroxide | 0.40 | 0.40 | |
| Beeswax | 5.00 | 5.00 | B |
| Carnauba Wax | 3.00 | 3.00 | |
| Pentaerythritol Tetrabehenate | 6.00 | 6.00 | |
| Lanolin | 2.50 | 2.50 | |
| Hydroxylated Lanolin | 1.00 | 1.00 | |
| n-Propylpara-hydroxybenzoate | 0.05 | 0.05 | |
| Stearic Acid | --- | 1.00 | |
| Black Iron Oxides[3] | 8.00 | 10.00 | C |
| Quaternium-15[4] | 0.20 | 0.20 | D |
| Water | 0.80 | 0.80 | |

[1] A copolymer of 2 or more monomers selected from acrylic acid, methacrylic acid or their simple esters, e.g., Rhoplex K-3, the tradename for such a copolymer available from Rohm and Hass Co., Inc., Philadelphia, Pa., U.S.A.

[2] One part by weight of solid acrylates copolymer, e.g., Carboset 525[a] and one part by weight of an about 30% solution of acrylates copolymer, e.g., (Carboset XL-28[b]).

[a] A tradename of a solid acrylic/acrylate copolymer available from B.F. Goodrich

[b] tradename of an about 30 weight % solution of acrylates copolymer

[3] Black iron oxides and other inorganic pigments.

[4] The quaternary ammonium salt, N-(3-Chloroallyl)-Hexaminium Chloride

Heat, with mixing, the ingredients in Part A (except ammonium hydroxide) at a temperature of 75°-85°C; continue mixing until hydration is complete and a homogeneous dispension is formed. Add ammonium hydroxide. Add, with mixing, Part B to mixture so formed while maintaining temperature at 75°-85°C. Add C with mixing at 75°-85°C. Cool to 45°-50°C and added D while continuing mixing until homogeneous mixture of all ingredients is achieved.

EXAMPLE IX

LONG WEAR LIP REPAIR CREAM

| Ingredients | Parts by Wgt. | |
|---|---|---|
| Deionized Water | 59.25 | A |
| Carbomer 934P | 0.20 | |
| Glycerine | 10.0 | B |
| Methyl p-Hydroxybenzoate | 0.2 | |
| Cyclomethicone | 4.0 | C |
| Glyceryl Stearate SE[1] | 2.0 | D |
| Pentaerythritol Tetrabehenate | 8.0 | |
| Polawax | 5.0 | |
| Cetyl Alcohol | 1.0 | |
| Oleth-3 Phosphate | 1.5 | |
| Octyl Palmitate | 3.0 | |
| n-Propyl p-Hydroxybenzoate | 0.1 | |

-18-

| Ingredients | Parts by Wgt. | |
|---|---|---|
| Triethanolamine | 0.7 | E |
| Deionized Water | 1.0 | |
| Disodium Ethylene Diamine | | F |
| Tetraacetic Acid | 0.1 | |
| Imidazolidinyl Urea | 0.4 | |
| Deionized Water | 2.0 | |
| Saccharide Isomerate | 1.0 | G |
| Reticulin (The Protein) | 0.5 | |
| Vitamin E | 0.05 | |

[1] A self-emulsifying grade of the ester of glycerine and stearic acid, which contains some sodium and/or potassium stearate.

Prepare by dispersing Carbomer 934P in water (Part A) until complete hydration is achieved. Heat the mixture so formed to 75°C and add a mixture of glycerine and methyl p-hydroxybenzoate (Part B). Add cyclomethicone (Part C) to the oil phase (Part D) prior to emulsification, (a temperature of 80°C). Heat, with mixing, all the ingredients (Parts C and D) to 85°C until a uniform dispersion is achieved. Add the oil phase (Parts C and D) to water phase (Parts A and B) and mix as the temperature is lowered to 70°C. Add Part E and continue mixing as temperature is lowered to 60°C. Add Part F and continue mixing. Cool the mixture to 40°C and add thereto Part G.

## COMPARATIVE EXAMPLE X

### LIP REPAIR CREAM

| Ingredients | Parts by Wgt. | |
|---|---|---|
| Deionized Water | 66.75 | A |
| Carbomer 934P | 0.2 | |
| Glycerine | 4.0 | B |
| Methyl p-Hydroxybenzoate | 0.2 | |
| Cyclomethicone | 4.0 | C |
| Pentaerythritol Tetrastearate | 8.0 | D |
| Glyceryl Stearate SE | 3.5 | |
| Polawax | 5.0 | |
| Cetyl Alcohol | 1.0 | |
| Oleth-3 Phosphate | 1.5 | |
| Octyl Palmitate | 3.0 | |
| n-Propyl p-Hydroxybenzoate | 0.1 | |
| Triethanolamine | 0.7 | E |
| Deionized Water | 1.0 | |
| Disodium Ethylene Diamine | | F |
| Tetraacetic Acid | 0.1 | |
| Imidazolidinyl Urea | 0.4 | |
| Deionized Water | 2.0 | |
| Saccharide Isomerate | 1.0 | G |
| Reticulin (The Protein) | 0.5 | |
| Vitamin E | 0.05 | |

[1] A self-emulsifying grade of the ester of glycerine and stearic acid, which contains some sodium and/or potassium stearate.

Prepare by following the procedure of Example IX except that an equivalent quantity of pentaerythritol tetrastearate is substituted for pentaerythritol tetrabehenate and the amounts of water, glycerine and glyceryl stearate SE are changed to achieve a better

-20-

consistency. The lip repair cream of this Example was tested and found to be inferior to that of Example IX.

## EXAMPLE XI
### LONG WEAR FOUNDATION

| Ingredients | Parts by Wgt. | |
|---|---|---|
| Deionized Water | 41.55 | A |
| Magnesium Aluminum Silicate | 1.00 | |
| Propylene Glycol | 5.00 | B |
| Alcolec 4135[1] | 1.00 | |
| A mixture of Isopropyl, Butyl and Isobutyl p-Hydroxybenzoates | 0.50 | |
| Propylene Glycol | 2.00 | C |
| Carboxymethylcellulose Gum | 0.20 | |
| Triethanolamine | 0.75 | D |
| Light Ivory Color Mix[2] | 12.50 | E |
| Deionized Water | 12.80 | |
| Dimethicone | 0.10 | F |
| Disodium Oleamide PEG-2 Sulfosuccinate[3] | 1.00 | |
| Stearic Acid | 1.50 | G |
| Pentaerythritol Tetrabehenate | 2.00 | |
| Corn Starch | 3.00 | |
| A mixture of ($C_{12}$-$C_{15}$) Alkyl Benzoates | 4.00 | |
| Propylene Glycol Dioctanoate | 5.00 | |
| Cyclomethicone | 4.00 | H |
| Imidazolidinyl Urea | 0.50 | I |
| Deionized Water | 1.50 | |
| Fragrance | 0.10 | J |

[1] A mixture of Lecithin and Polysorbate 20[a] and sorbitan laurate and propylene glycol stearate and propylene glycol

laurate available from American Lecithin Co., Inc., Atlanta
Georgia, U.S.A.

(a) A mixture of laurate esters of sorbitol and
sorbitol anhydrides, consisting predominantly of the
monoester, condensed with approximately 20 moles of ethylene
oxide.

[2] A mixture of red and yellow iron oxides, talc, titanium
dioxide, ultramarine blue and kaolin.

[3] The disodium salt of the monooleyl amide of the
diethylene glycol half-ester of sulfosuccinic acid
anhydride, available from Witco Chemical Corp., Organics
Div., N.Y., N.Y., U.S.A.

Prepare by heating the ingredients in Part A, with
mixing, to a temperature of 80°C. Add the ingredients of
Parts B, C and D and continue mixing until a uniform
dispersion is achieved. Add the color mix of Part E. Pass
the mixture so formed through a colloid mill and add water
of Part E. Add Part F and reheat the so-formed mixture to
80°C. In a steam-jacketed kettle equipped with an agitator,
heat the oil phase (Part G) to 80°C. Add, with mixing, the
oil phase and Part H to the mixture of Parts A to F while
maintaining the temperature at 80°C. Cool mixture so formed
to 60-66°C. Cool mixture so formed to 50-55°C and add Parts
I and J.

-22-

CLAIMS

1.      A composition adaptable for use as a long wear cosmetic base or as a long wear cosmetic comprising from about 2 to about 25 weight percent of a pentaerythritol tetra($C_{20}$-$C_{24}$) aliphatic hydrocarbon carboxylate in a conventional cosmetic or cosmetic base.

2.      The cosmetic composition of claim 1 wherein the aliphatic hydrocarbon carboxylate groups have straight chains.

3.      The cosmetic composition of claim 2 wherein the aliphatic hydrocarbon carboxylate groups are alkanoates.

4.      The cosmetic composition of claim 3 wherein said pentaerythritol alkanoate is pentaerythritol tetrabehenate.

5.      The cosmetic composition of any one of claims 1 to 5 which contains about 5 to about 20 weight percent of pentaerythritol tetra($C_{20}$-$C_{24}$) aliphatic hydrocarbon carboxylate.

6.      A composition of any one of claims 1 to 5 adapted for use as a long wear cosmetic base.

7.      A composition of anyone of claims 1 to 5 adapted for use as a long wear eye cream, long wear lip cream, long wear lipstick, long wear eye wear stick, long wear eyeliner, long wear mascara or long wear foundation.

8.      A method of extending the effective wear time of a cosmetic comprising admixing pentaerythritol tetra ($C_{20}$-$C_{24}$) aliphatic hydrocarbon carboxylate with a conventional cosmetic or cosmetic base so that the resulting composition contains from about 2 to about 25 weight percent pentaerythritol tetra ($C_{20}$-$C_{24}$) aliphatic hydrocarbon carboxylate.

9.        The method of claim 8 wherein the conventional cosmetic base in an eye cream, lip cream, lipstick, eye wear stick, eyeliner, mascara, or foundation.

10.        The method of claims 8 or 9 wherein the aliphatic hydrocarbon carboxylate groups have straight chains.

11.        The method of claims 8 or 9 wherein the aliphatic hydrocarbon carboxylates are alkanoates.

12.        The method of claim 11 wherein the pentaerythritol alkanoate is pentaerythritol tetrabehenate.